# EUROPÄISCHE PATENTSCHRIFT

(11) **EP 2 538 804 B1**
(45) Veröffentlichungstag und Bekanntmachung des Hinweises auf die Patenterteilung: **15.04.2015**
(21) Anmeldenummer: 12701087.4
(22) Anmeldetag: 11.01.2012
(51) Int. Cl.: A23L 1/30, A23L 2/52, A23L 2/68, A61P 5/26, A61P 15/10, A61K 31/215, A61K 31/19

(54) **GETRÄNK**
BEVERAGE
BOISSON

(30) Priorität: 12.01.2011 AT 372011
(43) Veröffentlichungstag der Anmeldung: 02.01.2013
(73) Patentinhaber: Daxer, Albert, 4073 Wilhering (AT)
(72) Erfinder: Daxer, Albert, 4073 Wilhering (AT)
(74) Vertreter: KLIMENT & HENHAPEL
(86) Internationale Anmeldenummer: PCT/EP2012/050380
(87) Internationale Veröffentlichungsnummer: WO 2012/095461

(56) Entgegenhaltungen:
- EP-A1- 2 008 691
- CA-A1- 2 530 216
- DE-U1- 29 709 474
- FR-A1- 2 124 399
- US-A1- 2003 203 004
- OMURA HISASHI ET AL: "Identification of feeding attractants in oak sap for adults of two nymphalid butterflies, Kaniska canace and Vanessa indica", PHYSIOLOGICAL ENTOMOLOGY, Bd. 25, Nr. 3, September 2000 (2000-09), Seiten 281-287, XP002674143, ISSN: 0307-6962
- WHELAN R J ET AL: "Short-chain carboxylic acids from gray catbird (Dumetella carolinensis) uropygial secretions vary with testosterone levels and photoperiod", COMPARATIVE BIOCHEMISTRY AND PHYSIOLOGY. PART B, BIOCHEMISTRYAND MOLECULAR BIOLOGY, ELSEVIER,OXFORD, GB, Bd. 156, Nr. 3, 1. Juli 2010 (2010-07-01), Seiten 183-188, XP027045064, ISSN: 1096-4959 [gefunden am 2010-05-11]

## Beschreibung

### GEBIET DER ERFINDUNG

Die Erfindung betrifft ein Getränk, enthaltend Wasser und zumindest eine Kombination der Inhaltsstoffe Essigsäure, Propansäure und Butansäure. Die Erfindung betrifft auch eine entsprechende Zusammensetzung zur oralen Einnahme sowie eine Zusammensetzung zur Herstellung eines erfindungsgemäßen Getränks.

### STAND DER TECHNIK

Die FR 2124399 A beschreibt eine Kombination von den eingangs genannten Fettsäuren, die bei Frauen, die orale Kontrazeptiva einnehmen, vorwiegend auf die Haut bzw. oberflächlich (topisch) aufgebracht wird. Durch die Duftwirkung (Abdampfung der Fettsäuren) entfalten die Fettsäuren am Riechorgan des Mannes eine sexuell stimulierende Wirkung, allerdings mit dem Nachteil, dass damit ein sehr unangenehmer Geruch verbunden sein kann. Eine Verwendung als Getränk ist nicht angeregt.

Es ist z.B. aus der US 2003/0203004 A1 ein Getränk bekannt, welches Essigsäure, Propansäure und Butansäure (Buttersäure) zusammen mit langkettigen Fettsäuren enthält und mit welchem eine Reduzierung des Körpergewichtes zu diätischen Zwecken erreicht werden soll.

Außerdem ist aus der CA 2530216 A1 bekannt, dass Essigsäure und Propansäure in einem Getränk zur Senkung des Blut-pH-Wertes verwendet werden können, sowie um die Auswirkung von Insektenstichen zu lindern oder um ganz allgemein ein entzündungshemmendes Milieu zu schaffen.

### DARSTELLUNG DER ERFINDUNG

Weder die US 2003/0203004 A1 noch die CA 2530216 A1 ist geeignet, den Testosteronspiegel des Menschen zu steigern bzw. die anderen erfindungsgemäßen Wirkungen zu erzeugen. Vielmehr beschreibt die US 2003/0203004 A1 einen praktisch gegenteiligen Effekt, indem es diätisch wirksam antianabol wirkt. Die Absicht der gegenständlichen Erfindung ist es allerdings u.a. einen anabolen Effekt durch Steigerung des natürlichen Testosteronspiegels zu erreichen und so quasi substanzaufbauend zu wirken. Im Übrigen ist die Fähigkeit zum konstant Halten des Blut-pH-Wertes eine wesentliche physiologische Funktion eines gesunden Körpers. Im Allgemeinen wird der pH-Wert des Blutes in sehr engen Grenzen durch die Funktion der Niere konstant bei 7,4 gehalten (maximal zwischen 7,35 und 7,45) sodass die Absicht der CA 2530216 A1 durch Zugabe von Säuren in den Nahrungsprozess den Blut-pH-Wert relevant zu senken in einem gesunden Körper gar nicht erreicht werden kann. Sollte es, durch welche Gründe auch immer, trotzdem eintreten, dass der Blut-pH-Wert außerhalb dieser engen Grenzen relevant in den sauren Bereich verschoben wird, kann durch diese künstlich herbeigeführte Acidose des Körpers sehr wahrscheinlich ein lebensgefährlicher Zustand ausgelöst werden.

Es ist eine Aufgabe der gegenständlichen Erfindung ein Getränk vorzuschlagen, das durch eine geeignete Kombination der Inhaltstoffe Essigsäure, Propansäure, Butansäure mit anderen Inhaltsstoffen eine natürliche Steigerung der Testosteronausschüttung bzw. generell der Androgenausschüttung einschließlich Androstan und DHEA beim Menschen, insbesondere beim Mann, bewirkt.

Die Aufgabe wird durch ein Getränk gemäß Anspruch 1 gelöst, das bereits Wasser und zumindest eine Kombination der Inhaltsstoffe Essigsäure, Propansäure und Butansäure enthält, wobei zur Steigerung der Testosteronausschüttung (bzw. generell der Androgenausschüttung) beim Menschen zusätzlich die Inhaltsstoffe Methylpropansäure und/oder Methylbutansäure enthalten sind, sowie als Zusatzstoff zur Maskierung des Geruchs der Inhaltsstoffe Zucker und/oder Süßstoff und/oder Alkohol enthalten ist. Das Getränk enthält also entweder die Kombination der Inhaltsstoffe Essigsäure, Propansäure, Butansäure, Methylpropansäure oder die Kombination der Inhaltsstoffe Essigsäure, Propansäure, Butansäure, Methylbutansäure oder die Kombination aller fünf Inhaltsstoffe Essigsäure, Propansäure, Butansäure, Methylpropansäure, Methylbutansäure. Die Kombination aller fünf Inhaltsstoffe hat sich als besonders wirksam erwiesen, weil sie der Zusammensetzung des Vaginalsekrets der empfängnisbereiten Frau besonders ähnlich ist und folglich besonders stimulierend auf die Testosteronproduktion (Androgenproduktion) beim Mann wirkt.

Die Erfindung betrifft also aufgrund ihrer Fähigkeit zur Steigerung der Testosteronausschüttung ein Getränk zur Steigerung der menschlichen Leistungsfähigkeit. Es ist bekannt, dass Testosteron die Leistungsfähigkeit des Menschen steigern kann. Es handelt sich beim Testosteron um ein männliches Geschlechtshormon, das natürlich im Körper vorkommt und eine anabole Wirkung hat. Leistungssteigernde synthetische Anabolika sind oft künstliche Derivate oder Analoga des Testosterons. Diese haben oft schwere, manchmal lebensbedrohliche Nebenwirkungen, wenn sie unkontrolliert zugeführt werden.

Die Erfindung verwendet eine Kombination der Inhaltsstoffe Essigsäure, Propansäure, Butansäure, Methylpropansäure, Methylbutansäure als Zusatz in einem Getränk (z.B. in sogenannten Energy Drinks) zur Steigerung des körpereigenen Testosterongehaltes und zur Erhöhung der Leistungsfähigkeit von Menschen und insbesondere von Sportlern. Es ist auch geeignet, die Libido von Männern durch Erhöhung des Testosteronspiegels zu steigern.

Da diese Inhaltsstoffe in dieser Kombination jedoch äußerst unangenehm riechen und der Geruchseffekt praktisch nicht zumutbar ist, wird die Applikation in Form eines Getränkes vorgeschlagen, das als weitere geeignete Zusatzstoffe Zucker und/oder Süßstoff und/oder Alkohol enthält, um sicherzustellen, dass im Bereich des olfaktorischen Rezeptorsystems der gewünschte Effekt ausgelöst werden kann, ohne von einer direkten Geruchsempfindung oder ohne von einer negativen Geruchsempfindung begleitet zu sein. Das Olfaktorische System besteht aus Riechzellen (Rezeptorzellen), die an der Innenwand (Schleimhaut) der Nasenhöhle (oberhalb der oberen Nasenmuschel) lokalisiert sind. Diese sind von einem Sekret (Schleim) bedeckt, in das die Zilien (Nervenhaare), die geeignete Rezeptorproteine aufweisen, teilweise eintauchen. Indem die Aufnahme der Inhalts- und Zusatzstoffe systemisch erfolgt und sie in der Weise ausgewogen und aufeinander abgestimmt sind, dass eine direkter Geruchseffekt ausgeschlossen bzw. nicht relevant ist, jedoch ein Wirkspiegel durch Sekretion der Inhalts- und Zusatzstoffe in das Nasenschleimhautsekret erzeugt wird, der zwar den gewünschten Hormoneffekt (z.B. Steigerung des Testosterons), aber keine unangenehme Geruchsempfindung auslöst, kann eine Aktivierung der Rezeptorproteine erfolgen ohne eine hohe Duftkonzentration in der Nasenhöhle erzeugen zu müssen. Die Aktivierung der gewünschten Reaktion erfolgt dadurch praktisch von innen. Ein Effekt (Aktivierung) ist dadurch auch direkt an den Rezeptorzellen (Zellwand) möglich. Dies erfolgt dann zumindest zum Teil unter Umgehung der Riechhärchen (Zilien). Da die Resorbtion und Sekretion der verschiedenen Inhalts- und Zusatzstoffe unterschiedlich ist, kann es sinnvoll sein, die Relativkonzentrationen der Stoffe bei der Zuführung (im Getränk, im Getränkepulver, etc.) unterschiedlich zu der effektiven Konzentration im Endorgan (Nasenschleimhaut, Sekret, Blut, Interzellulärsubstanz, Zellenoberfläche, Schweiß, Körperoberfläche, Körperhöhlen, etc.) zu gestalten.

Durch das erfindungsgemäße Getränk wird eine direkte (bewusste) Geruchswirkung der Substanzen am Riechorgan des Menschen ausgeschaltet (maskiert). Vielmehr wird durch Resorption, selektive oder generelle Verteilung im Körper und Sekretion bzw. Ausscheidung der Substanzen oder deren Vorstufen an inneren oder äußeren Körperoberflächen eine indirekte bzw. unbewusste Wirkung am Endorgan (Olfaktorisches System, Limbisches System, chemische Rezeptoren und Nervenendigungen und -fasern, Leydigsche Zwischenzellen und Hoden, etc.) erreicht. Bei Frauen führt es nicht zu einer Steigerung des Testosteronspiegels.

Eine weitere (oder zusätzliche) Aufgabe ist die Erzeugung eines isotonischen Getränkes ohne eine bedeutende Zuckerbelastung (Kohlehydratbelastung) für den Körper. In sogenannten isotonischen Getränken wird der osmotische Effekt zum überwiegenden Teil durch den Kohlehydratgehalt erzielt.

Durch die oben beschriebene Auslösung des Hormoneffektes kann auch ein positiver Rückkopplungskreislauf in Gang gesetzt werden, der bei der Applikation, die eine direkte Geruchswirkung einschließt, etwa durch Versprühen der Inhaltsstoffe auf die Haut, unterbleibt. Durch den unangenehmen Geruch der Substanzen würde der Hormoneffekt durch Vermeidungsmechanismen negativ beeinflusst. Bei geeigneter Zuführung der Substanzen (Stoffe), etwa in maskierter Form und/oder in Kombination mit anderen Substanzen in einem geeigneten Getränk, die den direkten Geruchseffekt abschirmen, kann auch eine Modulation des Effektes durch den aktuellen Hormonstatus (z.B. Testosteronkonzentration) selbst erfolgen. Dieses Abschirmen kann durch geeignete molekulare Verbindungen der Stoffe untereinander oder geruchsmäßige Abschirmung durch isolierte Zusatzsubstanzen erfolgen.

Durch die Applikation der Inhaltstoffe und Konsumation des entsprechenden Getränkes kann auch eine bestimmte Beeinflussung bzw. Modifikation der Stimmungs- und Wahrnehmungslage des das Getränk Trinkenden oder eines sich in seiner unmittelbaren Umgebung befindlichen Menschen erreicht werden. Je nach Ausgangslage für Stimmung und Wahrnehmung und je nach Konzentration und Zusammensetzung der Inhaltstoffe können dabei unterschiedliche Stimulationen auf die menschliche Psyche und Physis ausgelöst werden. Zu diesen gehören, ohne Anspruch auf Vollständigkeit, Hebung der Energie, Hebung des natürlichen Selbstbewusstseins, Hebung der Lebensfreude, Hebung der sexuellen Anziehungskraft, Senkung psychischer zwischenmenschlicher Barrieren, Hebung der intellektuellen Auffassungsgabe, Hebung der sexuellen Potenz, Hebung der Libido und des gefühlsmäßigen Empfindens allgemein, Senkung der Orgasmusschwelle bei der Frau, Erhöhung der sexuellen Ausdauer beim Mann, etc. Das Getränk erhöht auch die (sportliche) Leistungsfähigkeit, Konzentration und beeinflusst beim Mann die männlichen Verhaltensweisen positiv z.T. durch Aktivierung der natürlichen Testosteronbildung beim Mann. Bei der Frau, die das erfindungsgemäße Getränk trinkt, wirkt das Getränk in der Form, dass es die (sinnliche) Wahrnehmung als Frau beim Mann erhöht und so indirekt positive Effekte auf die Attraktivität der Frau in den Augen des Mannes ausübt.

Der pH-Wert des Getränkes sollte dabei vorzugsweise zwischen 2,0 und 7,5 liegen, idealer Weise zwischen 2,5 und 7,0. Dabei ist ein pH-Wert von kleiner als 5, insbesondere kleiner als 4, sinnvoll, wenn das Getränk ein Erfrischungsgetränk (Softdrink) sein soll. Erfrischungsgetränke sind meist kohlensäurehaltig und süßsauer aromatisiert. Unter Erfrischungsgetränk fallen Getränke wie Fruchtsaftgetränke, Limonaden und Brausen.

Besonders günstige Verhältnisse können in besonderen Ausführungsformen durch einen pH-Wert des Getränkes unter 3 (zwischen 2 und 3) erreicht werden. Insbesondere die Maskierung der direkten Geruchswirkung, die Verteilung und Sekretion kann dadurch, in Abhängigkeit von der exakten Zusammensetzung, verbessert werden.

Als Inhaltsstoffe des Getränkes im Sinne der gegenständlichen Erfindung (Gruppe 1) gelten Essigsäure, Propansäure, Butansäure, Methylpropansäure, Methylbutansäure.

Als Zusatzstoffe des Getränkes im Sinne der gegenständlichen Erfindung (Inhaltsstoffe der Gruppe 2) gelten alle anderen genannten Substanzen. Grundsätzlich können alle Substanzen, Inhaltsstoffe und Zusatzstoffe sowie alle Ausführungsformen geeignet zu neuen Ausführungsformen kombiniert werden.

Zusatzstoffe können z.B. sein: Wasser, Zucker (bis 150 g pro Liter), Mineralien, Vitamine und Proteine; Fruchtsäfte und Aromen; anregende Stoffe wie Taurin (bis zu 20 g pro Liter) oder Coffein (bis zu 1,5 g pro Liter) oder Teein.

Zur Erzielung eines isotonen Effektes sollte die Osmolarität um 300 mosmol pro Liter (bis 450 mmol/kg) betragen, was ca. 0,7 Mpascal entspricht. Ideal sind 308 mosmol pro Liter (isoton) entprechend der Osmolarität des Blutes. Vorzugsweise liegt der Wert zwischen 290 und 300 mmol/kg oder 290 mmol/kg +/- 15%. In einer besonderen Ausführungsform sollten noch mindestens 1000 mg Mineralstoffe pro Liter und ein bestimmter Gehalt an Na, K, Ca, Mg und Cl vorhanden sein.

In einer weiteren Ausführungsform können Mengen- und Makroelemente > 50 mg pro kg Körpergewicht (z.B. Ca, Cl, K, Mg, Phosphor, Schwefel, Na) und/oder Spurenelemente (Mikroelemente) < 50 mg pro kg Körpergewicht (z.B. esentielle Spurenelemente: Chrom, Cobalt, Eisen, Fluor, Jod, Cu, Mangan, Molybdän, Selen, Silizium, Vanadium, Zink; Arsen, Bor, Rubidium, Zinn), Baustoffe: Natrium, Kalium, Calcium, Magnesium und Phosphor sowie Reglerstoffe: Eisen, Jod, Kupfer als Zusatzstoffe vorhanden sein.

Das Getränk kann durch Eiweißzusätze z.B. Holunderblütendolden, Zitrone, Honig, z.B. andere Früchte wie Melonen (z.B. 600 g), 400 g Hibiskusblüten (Blüten), z.B. andere Zutaten wie Eis, Sorbet (z.B. 200 g), Joghurt (z.B. 250 g), Orangen bzw. Orangensaft, Milch (z.B. 500 ml), Zucker, Eigelb, Vanillezucker, Kaffee (Pulver, löslich), Kakaopulver, Bier, Rum, Gin, Birnen bzw. Birnensaft, Ingwer (z.B. 20 g, kandiert, klein geschnitten, Pulver, löslich), Zwiebel, Knoblauch angereichert sein.

Bei dem Getränk bzw. trinkbaren Flüssigkeit handelt es sich um eine Zusammensetzung bestehend aus einer flüssigen Grundsubstanz die die Inhaltsstoffe der Gruppe 1 und zumindest einen Inhaltsstoff der Gruppe 2 enthält.

Bei den Getränken oder trinkbaren Flüssigkeiten kann es sich um jede beliebige handeln, z.B. Tee, Kaffee, Milchgetränke, Limonaden, Colagetränke, Fruchtsäfte, Fruchtsaftkonzentrate, Biere, Liköre, Weine, nichtalkoholische oder alkoholische Getränke, Energy-Drinks, etc.

Weitere Inhaltsstoffe der Gruppe 2 können z.B. je nach Anwendung (Energydrink, Sportgetränk, Erfrischungsgetränk, alkoholisches Getränk, etc.) sein: Trinkwasser, natürliches Mineralwasser, Quellwasser, Tafelwasser, Kohlensäure, Zucker (z.B. Rübenzucker, Traubenzucker, Rohrzucker, Invertzucker), Zuckerersatzstoff, Süßstoff (z.B. Steviaglycoside (E960), Acesulfam, Aspartam, Aspartan-Acesulfam-Salz, Cyclamat, Saccharin, Sucralose, Thaumatin, Neohesperidin, Steviosid, Neotam; Alitam, Brazzein, Hernandulcin, Lugdunam, Monellin, Pentadin), Aromaextrakte, natürliche oder künstliche Aromastoffe, Citronensäure, Fruchtsaft, Fruchtsaftkonzentrat, Fruchtmark, Fruchtmarkkonzentrat, Zuckerkulör (z.B. E150, E150a, b, c, d), Koffein, Beta-Carotin, Riboflavin, Auszüge aus Ingwerwurzel, Bitterstoffe (z.B. Chinin), Alkohol (z.B. Äthylalkohol), Magnesium, Calcium, Phosphat, Eisen, Zink, Natrium, Kalium, Folsäure, Harnsäure, Proteine, Aminosäuren, Kohlehydrate, Vitamine (z.B. Vitamin A, B1, B2, B6, C, D, E), Fettsäuren (gesättigt oder ungesättigt), Geschmackstoffe oder jeder in der Genussmittelindustrie bekannte Zusatzstoff von Getränken jeglicher Art.

In einer besonderen Ausführungsform kann z.B. ein Steviaglycosid (E960) beliebig dosiert werden, aber z.B. mit 10 mg bis 500 mg pro Liter in Kindergetränken und 50 mg bis 2000 mg pro Liter in Erwachsenengetränken zusammen, Süßwert 300 mal stärker als Zucker. Z. B. kann die Steviadosierung gemäß bis 4 mg pro kg Körpergewicht (am Tag) bzw. bis zu ideal 12 mg pro kg Körpergewicht erfolgen. Beispielsweise kann also Stevia mit vorzugsweise 100 bis 500 mg pro Liter bzw. in einer besonderen Ausführungsform mit 250 mg bis 300 mg pro Liter dosiert werden.

In einer besonderen Ausführungsform (z.B. zur Entwässerung) kann die Osmolarität des Getränkes über 450 mmol/kg sein. In einer anderen Ausführungsform (z.B. zur Rehydrierung) soll die Osmolarität ca. 300 mmol/kg nicht bzw. nicht wesentlich übersteigen. Vorzugsweise ist es in diesem Fall unter 300 mmol/kg bzw. idealerweise unter 100 mmol/kg.

In einer weiteren Ausführungsform sollte bei notwendiger Energiezufuhr (z.B. Sport) der Kohlehydratgehalt (z.B. Glukose, Saccharose) über 3% (am Gewicht oder Volumen) des Getränkes sein, in besonderen Fällen sogar über 8% (bis zu 15%) betragen.

In der erfindungsgemäßen, Ausführungsvariante ist die relative Zusammensetzung der Inhalsstoffe der Gruppe 1 wie folgt:
Essigsäure 75% bis 85%
Propansäure 10% bis 20%
Butansäure bis 10%
Methylpropansäure bis 5%
Methylbutansäure bis 5%

Oder insbesondere
Essigsäure 75% bis 80%
Propansäure 10% bis 15%
Butansäure bis 7%
Methylpropansäure bis 1%
Methylbutansäure bis 5%

Oder
Essigsäure 75% bis 85%
Propansäure 10% bis 20%
Butansäure bis 5%
Methylpropansäure bis 1 %
Methylbutansäure bis 5%

Also z.B.
Essigsäure 78,38%
Propansäure 13,21%
Butansäure 4,94%
Methylpropansäure 0,76%
Methylbutansäure 2,14%

Je nach Ausprägung der Wirkung angewendet auf Mann oder Frau, Alter, Aktivität, etc. kann die Kombination der Inhaltsstoffe und die Kombination der Inhaltsstoffe mit Zusatzstoffen unterschiedlich sein.

Dabei sollte der Volums- oder Gewichtsanteil der Summe der Inhaltstoffe der Gruppe 1 oder zumindest eines der Inhaltstoffe der Gruppe 1 an einer Mischung mit der flüssigen Grundsubstanz möglichst 5% nicht übersteigen. Vorzugsweise ist er kleiner als 1% und idealer Weise kleiner als 0,1% oder je nach sonstigen Inhaltsstoffen, etwa der Gruppe 1, auch kleiner als 0,01%. Der Volums- oder Gewichtsanteil der Summe der Inhaltstoffe der Gruppe 1 oder zumindest eines der Inhaltstoffe der Gruppe 1 an einer Mischung mit der flüssigen Grundsubstanz kann aber auch beliebig sein.

In besonderen Anwendungen, wie z.B. bei der Rehydrierung mit reichlich Flüssigkeitszufuhr, etwa nach Sport, kann der Volums- oder Gewichtsanteil der Summe der Inhaltstoffe der Gruppe 1 oder zumindest eines der Inhaltstoffe der Gruppe 1 an einer Mischung mit der flüssigen Grundsubstanz zwischen 1 % und 5%, zwischen 0,1% und 1% oder zwischen 0,01% und 0,1% betragen. Etwa 0,08%, 0,04% oder auch 0,01% oder kleiner bzw. 0,005% oder kleiner.

Es ist auch möglich, dass die Inhaltstoffe der Gruppe 1 in Form einer Tablette oder einer anderen festen oder pulverförmigen oder gelartigen Form verabreicht wird. Dann muss die Konzentration in dieser Darreichungsform so gewählt werden, dass die beschriebenen Konzentrationsverhältnisse beim Auflösen in der jeweiligen Körperflüssigkeit (z.B. Speichel) den oben angegebenen Werten entsprechen. Bei Verabreichung zum Auflösen in Flüssigkeit, z.B. in Form einer Brausetablette o.ä., sollte die Konzentration in der daraus gewonnenen Flüssigkeit den oben angegebenen Werten folgen.

In einer besonderen Ausführungsform ist die Grundsubstanz des Getränkes derart dass 1 Liter Wasser 9g NaCl (154 mmol/INa und 154 mmol/l Cl) eine Osmolarität von 309 mosm/l und einen pH-Wert von 5,5 bis 7 besitzt. Dazu werden dann die Inhaltsstoffe der Gruppe 1 gegeben, die dann den angegebenen pH-Wert und die Osmolarität im fertigen Getränk verändern.

Bei weiteren Ausführungsformen besonders geeignet zur Volumssubstiution oder Abpufferung des Blut-pH-Wertes z.B. bei Sport wird die Grundsubstanz (vor Zugabe der Inhaltsstoffe der Gruppe 1) wie folgt ausgeführt, zu der dann noch die Inhaltsstoffe der Gruppe 1 kommen:
1.Na 147 (140 - 150) mmol/l, K 4 (bis 5) mmol/l, Ca 2,2 (bis 5) mmol/l, Cl 156 (150 - 160) mmol/l mit einer Osmolarität von 309 (290 - 320) mosm/l.
2. Na 125 - 134 mmol/l, K 4,0 - 5,4 mmol/l, Ca 0,9 - 2 mmol/l, Cl 106 -117 mmol/l, Laktat 25 - 31 mmol/l mit einer Osmolarität von 262 - 293 mosm/l.
3.Na 130 (125 - 134) mmol/l, K 5,4 (5 - 6) mmol/l, Ca 0,9 (bis 2) mmol/l, Cl 112 (106 - 117) mmol/l, Acetat 27 (20 - 35) mmol/l mit einer Osmolarität von 250 - 300 mosm/l.

Besonders gute Ergebnisse hinsichtlich Maskierung der direkten Geruchswirkung, hinsichtlich Resorption, Verteilung und Sekretion können je nach Anwendung durch folgende Ausführungsformen erreicht werden:

Bei einem 250 ml Getränk:
25 mg Fettsäuren der Gruppe 1 (10 mg bis 200 mg)
75 mg Steviaglycoside (25 mg bis 200 mg)
75 mg Koffein (50 mg bis 150 mg)
1 g Taurin (0,5 g bis 2 g)
bzw.
25 mg Fettsäuren der Gruppe 1 (10 mg bis 200 mg)
25 g Zucker (10 g bis 30 g)
75 mg Koffein (50 mg bis 150 mg)
1 g Taurin (0,5 g bis 2g)
bzw.
25 mg Fettsäuren der Gruppe 1 (10 mg bis 200 mg)
50 mg Steviaglycoside (10 mg bis 100 mg)
15 g Zucker (10 g bis 20 g)
75 mg Koffein (50 mg bis 150 mg)
1 g Taurin (0,5 g bis 2 g)

Bei einem 50 ml Getränk:
5 mg Fettsäuren der Gruppe 1 (1 mg bis 20 mg)
15 mg Steviaglycoside (5 mg bis 30 mg)
70 mg Koffein (30 mg bis 100 mg)
800 mg Taurin (0,5 g bis 1,5 g)
bzw.
5 mg Fettsäuren der Gruppe 1 (1 mg bis 20 mg)
5 g Zucker (1 g bis 10 g)
70 mg Koffein (30 mg bis 100 mg)
800 mg Taurin (0,5 g bis 1,5 g)

Bei einem 500 ml Getränk:
50 mg Fettsäuren der Gruppe 1 (10 mg bis 200 mg)
150 mg Steviaglycoside (50 mg bis 300 mg)
80 mg Koffein (30 mg bis 100 mg)
1 g Taurin (0,5 g bis 1,5 g)

Besonders gute Verhältnisse werden erzielt, wenn die Menge an Steviaglycosiden etwa das 3-fache der Menge der Fettsäuren der Gruppe 1 (in mg) beträgt (ideal zwischen 0,1 und 10). Besonders gute Verhältnisse erreicht man auch, wenn der Zuckergehalt ca. das 1000-fache der Fettsäuren der Gruppe 1 beträgt (ideal zwischen 10 und 5000-fach), sowie wenn der Koffeingehalt um die 75 mg (ideal zwischen 50 mg und 200 mg) und/oder der Tauringehalt um 1 g (0,5 g bis 2 g) beträgt. Die Taurinkonzentration kann zwischen 0% und 1% (ideal zwischen 0,3 und 0,5%) und die Koffeinkonzentration kann zwischen 0% und 0,1% (ideal zwischen 0,03% und 0,05%) betragen. Somit kann die Menge an Taurin vorzugsweise das 30 bis 50 fache und/oder die Menge an Koffein das 3 bis 5-fache der Menge an Fettsäuren der Gruppe 1 betragen. Vorzugsweise ist auch die Menge an Steviaglykosiden und Koffein gleich (Stevia zu Koffein zwischen 0,2 und 3).

Die oral zugeführte Menge an Fettsäuren der Gruppe 1 durch das Getränk sollte, um eine brauchbare Wirkung zu erzielen, möglichst nicht weniger als 0,5 mg, besser mindestens 5 mg pro Tag, ideal jedoch 20 mg bis 30 mg pro Tag betragen. Eine Tagesmenge von 100 mg (maximal jedoch 1 g) sollte möglichst nicht überschritten werden.

Durch weitere geeignete Zusatzstoffe kann auch Einfluss auf die Viskosität des Getränkes genommen werden. So kann es für bestimmte Anwendungen (z.B. zur Verbesserung der Maskierung bei Vorhandensein von bestimmten Inhaltsstoffen) vorteilhaft sein, die Viskosität des Getränkes, die idealerweise 1 mPa.s (vorzugsweise zwischen 0,5 und 2) beträgt, zu erhöhen. Durch Beifügen von Säften (z.B. Fruchtsäften), Milch bzw. Milchderivaten, Ölen, Salzen oder anderen Substanzen kann die Viskosität bis auf 5, 10, 20, 50 oder 100 (in besonderen Fällen bis auf 1000 und darüber) gesteigert werden. In besonderen Fällen kann es notwendig sein, das Getränk mit speziellen Partikeln (Pflanzenstoffen, biologischen zellulären oder nichtzellulären Zusatzstoffen, etc) zu vermischen um eine Flüssigkeit, wo die Viskosität vom Fließzustand abhängt (nicht newtonsche Flüssigkeit) zu erzeugen. Dies hat insbesondere Vorteile, wenn bestimmte Zusatzstoffe, die im flüssigen Zustand sehr flüchtig sind, vor dem Abdampfen vor dem Genuss bewahrt werden sollen.

Durch geeignete Kombination der Inhaltsstoffe der Gruppe 1 mit Osmolarität, pH-Wert, Zucker oder Süßstoffgehalt, Mengen- oder Makroelemente, Spurenelemente, Vitaminen, Eiweißstoffen, Süße und Alkohol können z.B. bzgl. der direkten Wirkung an den Epithelzellen bzw. der Maskierung hinsichtlich eines direkten Geruchseffektes besonders gute Ergebnisse erzielt werden.

Die ausgewiesenen Inhaltstoffe der Gruppe 1, z.B. in den angegebenen Konzentrationen, bewirken besonders bei Frauen, dass es u.a. zu "Ausdünstungen" kommt, die den Testosteronspiegel eines männlichen Gegenübers erhöhen und das subjektive Empfinden der Attraktivität der Frau, die das Getränk aufgenommen hat, im Empfinden des männlichen Gegenübers steigern.

Grundsätzlich ist das erfindungsgemäße Getränk zur nicht-therapeutischen Anwendung vorgesehen, nämlich um durch die Einnahme des Getränks durch einen Menschen dessen Leistungsfähigkeit anzuheben.

Es ist jedoch auch denkbar, das erfindungsgemäße Getränk als Arzneimittel zu therapeutischen Zwecken zu nutzen, nämlich um einen erniedrigten Testosteronspiegel zu behandeln. Dies kann insbesondere bei Testosteron-Mangel-Erkrankungen (ADAM (Androgen Decline in the Aging Male), PADAM (Partial Androgen Decline in the aging male), Climacterium virile, LOH (Late onset Hypogonadism), TMS (Testosteron Mangel Syndrom), Hypogonadismus, etc.) zur Anwendung kommen. Eine Testosteron-Mangel-Erkrankungen führt beim erwachsenen Mann zur Abnahme der Libido, zur Abnahme der Vitalität, zur erektilen Dysfunktion, zur Reduktion der Muskelmasse, Reduktion der Knochendichte, Anämie und depressive Verstimmung. Durch die Behandlung mit dem erfindungsgemäßen Getränk bzw. in diesen Fällen generell als oral zuführbares Medikament sollen die Symptome bzw. deren Ursache gemildert bzw. bekämpft werden. Also z.B. die Libido gesteigert werden, die Vitalität erhöht werden, einer erektilen Dysfunktion entgegengewirkt werden, Muskeln aufgebaut werden, Knochendichte erhalten bzw. verbessert werden, die Anämie bekämpft werden und eine depressive Verstimmung behandelt werden und eine positive, lebensbejahende Stimmung induziert werden.

Je nachdem, welche Auswirkung behandelt werden soll, kann die relative Konzentration der Inhaltsstoffe Essigsäure, Propansäure, Butansäure, Methylpropansäure, Methylbutansäure zueinander gewählt werden:

Zur Steigerung der Libido:
Essigsäure 75% bis 85%
Propansäure 10% bis 20%
Butansäure 0,05% bis 10%
Methylpropansäure 0,1 % bis 5%
Methylbutansäure 0,1% bis 5%

Zum Entgegenwirken einer erektilen Dysfunktion:
Essigsäure 75% bis 85%
Propansäure 10% bis 15%
Butansäure 0,1 % bis 8%
Methylpropansäure 0,1% bis 5%
Methylbutansäure 0,1% bis 5%

Zum Muskelaufbau:
Essigsäure 75% bis 85%
Propansäure 10% bis 15%
Butansäure 0,1% bis 5%
Methylpropansäure 0,1 % bis 3%
Methylbutansäure 0,1 % bis 3%

Zur Erhaltung bzw. Verbesserung der Knochendichte:
Essigsäure 75% bis 85%
Propansäure 10% bis 15%
Butansäure 0,1 % bis 5%
Methylpropansäure 0,1% bis 1 %
Methylbutansäure 0,1% bis 3%

Auch ist es denkbar, die Fettsäuren der Gruppe 1 erfindungsgemäß als Getränk in einer homöopathischen Zubereitung zu verwenden. Dazu können etwa die Fettsäuren der Gruppe 1 in einer extrem niedrigen Konzentration vorhanden sein und trotzdem ihre Wirkung ohne direkte Beteiligung der bewussten Geruchswahrnehmung entfalten. So ist es denkbar, aus einem 1 %ig bis 10%igen Gemisch der Fettsäuren der Gruppe 1 mit Wasser oder Alkohol oder beidem eine entsprechende Verdünnungsreihe (Potenzierung) durch stufenweise Verdünnung von Verdünnungsschritt zu Verdünnungsschritt um den Faktor 10 der Fettsäuren der Gruppe 1 zur oralen Anwendung zu erhalten. So kann dies je nach Anwendung von D4 (= Verdünnung 1:10.000) bis D100 (= Verdünnung 1:10¹⁰⁰) erfolgen. Besonders gute Verhältnisse sind je nach Anwendung bei D5 bis D10 zu (= Verdünnung 1:100.000 bis 1:10.000.000.000) erwarten. Die homöopathische Zubereitung kann auch beliebige Zusatzstoffe der Gruppe 2 in beliebiger Konzentration enthalten.

Grundsätzlich sind alle Verbindungen, Dosierungen und Ausführungsbeispiele bzw. Teile davon mit allen Verbindungen, Dosierungen und Ausführungsbeispielen bzw. Teilen davon zu neuen Ausführungsbeispielen kombinierbar.

## Patentansprüche

1. Getränk für Menschen, enthaltend Wasser und zumindest eine Kombination der Inhaltsstoffe Essigsäure, Propansäure und Butansäure, **dadurch gekennzeichnet, dass** zusätzlich die Inhaltsstoffe Methylpropansäure und/oder Methylbutansäure enthalten sind,
wobei die relative Zusammensetzung der Inhaltsstoffe wie folgt ist:
Essigsäure 75% bis 85%,
Propansäure 10% bis 20%,
Butansäure bis 10%,
Methylpropansäure bis 5%,
Methylbutansäure bis 5%,
und wobei als Zusatzstoff zur Maskierung des Geruchs der Inhaltsstoffe Zucker und/oder Süßstoff und/oder Alkohol enthalten ist.

2. Getränk nach Anspruch 1, **dadurch gekennzeichnet, dass** Natrium, Kallum, Calcium, Magnesium, Phosphat, Eisen oder Zink-enthalten ist.

3. Getränk nach Anspruch 1 oder 2, **dadurch gekennzeichnet, dass** Kohlensäure enthalten ist.

4. Getränk nach einem der Ansprüche 1 bis 3, **dadurch gekennzeichnet, dass** die Summe der Inhaltsstoffe Essigsäure, Propansäure, Butansäure, Methylpropansäure, Methylbutansäure 6%, vorzugsweise 1%, Insbesondere 0,1%, des Volumens oder Gewichts des Getränkes nicht übersteigen.

5. Getränk nach einem der Ansprüche 1 bis 4, **dadurch gekennzeichnet, dass** es eine Osmolarität von 290 mmol/kg +/-15% aufweist.

6. Getränk nach einem der Ansprüche 1 bis 5, **dadurch gekennzeichnet, dass** es einen pH-Wert zwischen 2,0 und 7,5 aufweist.

7. Getränk nach einem der Ansprüche 1 bis 6, **dadurch gekennzeichnet, dass** Kohlehydrate enthalten sind.

8. Getränk nach einem der Ansprüche 1 bis 7, **dadurch gekennzeichnet, dass** Vitamine enthalten sind.

9. Getränk nach einem der Ansprüche 1 bis 8, **dadurch gekennzeichnet, dass** natürliche oder künstliche Aromastoffe enthalten sind.

10. Getränk nach einem der Ansprüche 1 bis 9, **dadurch gekennzeichnet, dass** Koffein und/oder Teein enthalten sind.

11. Getränk nach einem der Ansprüche 1 bis 10, **dadurch gekennzeichnet, dass** Taurin enthaltend ist.

12. Getränk nach einem der Ansprüche 1 bis 10, **dadurch gekennzeichnet**, dass-Mengenelemente, Makroelemente und/oder Spurenelemente enthalten sind.

13. Zusammensetzung in fester, pulverförmiger oder gelartiger Form, die zum Auflösen in einer Flüssigkeit formuliert ist, etwa Brausetabletten, zur Herstellung eines Getränks für Menschen nach einem der Ansprüche 1 bis 12, wobei die Zusammensetzung zumindest die Inhaltsstoffe Essigsäure, Propansäure und Butansäure enthält sowie zusätzlich die Inhaltsstoffe Methylpropansäure und/oder Methylbutansäure.

14. Verwendung eines Getränks nach einem der Ansprüche 1 bis 12 bzw. einer Zusammensetzung nach Anspruch 13 zur Steigerung der Testosteronausschüttung beim Menschen, insbesondere beim Mann.

15. Arzneimittel in Form eines Getränks nach einem der Ansprüche 1 bis 12 oder einer Zusammensetzung nach Anspruch 13 zur Vorbeugung und/oder Behandlung eines erniedrigten Testosteronspiegels.

## Claims

1. A beverage for humans, containing water and at least one combination of the ingredients of acetic acid, propanoic acid and butanoic acid, **characterized in that** the ingredients of methyl propanoic acid and/or methyl butanoic acid are additionally contained, wherein the relative composition of the ingredients is as follows:
acetic acid 75% to 85%
propanoic acid 10% to 20%
butanoic acid up to 10%
methyl propanoic acid up to 5%
methyl butanoic acid up to 5%,
and wherein sugar and/or sweetener and/or alcohol is contained as an additive for masking the smell of the ingredients.

2. A beverage according to claim 1, **characterized in that** it contains sodium, potassium, calcium, magnesium, phosphate, iron or zinc.

3. A beverage according to claim 1 or 2, **characterized in that** it contains carbonic acid.

4. A beverage according to one of the claims 1 to 3, **characterized in that** the sum total of the ingredients of acetic acid, propanoic acid, butanoic acid, methyl propanoic acid, methyl butanoic acid does not exceed 5%, preferably 1%, especially 0.1 %, of the volume or weight of the beverage.

5. A beverage according to one of the claims 1 to 4, **characterized in that** it has an osmolarity of 290 mmol/kg +/- 15%.

6. A beverage according to one of the claims 1 to 5, **characterized in that** it has a pH value of between 2.0 and 7.5.

7. A beverage according to one of the claims 1 to 6, **characterized in that** it contains carbohydrates.

8. A beverage according to one of the claims 1 to 7, **characterized in that** it contains vitamins.

9. A beverage according to one of the claims 1 to 8, **characterized in that** it contains natural or artificial flavouring agents.

10. A beverage according to one of the claims 1 to 9, **characterized in that** it contains caffeine and/or theine.

11. A beverage according to one of the claims 1 to 10, **characterized in that** it contains taurine.

12. A beverage according to one of the claims 1 to 10, **characterized in that** it contains abundant elements, macroelements and/or trace elements.

13. A composition in solid, powdery or gel-like form, which is formulated for dissolution in a liquid such as fizzy tablets, for producing a beverage for humans according to one of the claims 1 to 12, wherein the composition contains at least the ingredients of acetic acid, propanoic acid and butanoic acid, and additionally the ingredients of methyl propanoic acid and/or methyl butanoic acid.

14. The use of a beverage according to one of the claims of 1 to 12 or a composition according to claim 13 for increasing the testosterone release in humans, especially in the male.

15. A medication in form of a beverage according to one of the claims 1 to 12 or a composition according to claim 13 for preventing or treating a low testosterone level.

## Revendications

1. Boisson à usage humain, contenant de l'eau et au moins une combinaison des ingrédients : acide acétique, acide propanoïque et acide butanoïque, **caractérisée en ce qu'**elle contient également les ingrédients : acide méthylpropanoïque et/ou acide méthylpropanoïque, la composition relative des ingrédients étant la suivante :
acide acétique 75 % à 85 %,
acide propanoïque 10 % à 20 %,
acide butanoïque jusqu'à 10 %,
acide méthylpropanoïque jusqu'à 5%,
acide méthylbutanoïque jusqu'à 5 %,
et du sucre et/ou un édulcorant et/ou de l'alcool étant contenus pour masquer l'odeur des ingrédients.

2. Boisson selon la revendication 1, **caractérisée en ce qu'**elle contient du sodium, du potassium, du calcium, du magnésium, du phosphate, du fer ou du zinc.

3. Boisson selon la revendication 1 ou 2, **caractérisée en ce qu'**elle contient du gaz carbonique.

4. Boisson selon l'une des revendications 1 à 3, **caractérisée en ce que** la somme des ingrédients : acide acétique, acide propanoïque, acide butanoïque, acide méthylpropanoïque et acide méthylbutanoïque ne dépasse pas 5 %, de préférence 1 %, en particulier 0,1 %, du volume ou du poids de la boisson.

5. Boisson selon l'une des revendications 1 à 4, **caractérisée en ce qu'**elle présente une osmolarité de 290 mmol/kg ± 15 %.

6. Boisson selon l'une des revendications 1 à 5, **caractérisée en ce qu'**elle présente un pH compris entre 2,0 et 7,5.

7. Boisson selon l'une des revendications 1 à 6, **caractérisée en ce qu'**elle contient des hydrates de carbone.

8. Boisson selon l'une des revendications 1 à 7, **caractérisée en ce qu'**elle contient des vitamines.

9. Boisson selon l'une des revendications 1 à 8, **caractérisée en ce qu'**elle contient des aromes naturels ou artificiels.

10. Boisson selon l'une des revendications 1 à 9, **caractérisée en ce qu'**elle contient de la caféine et/ou de la théine.

11. Boisson selon l'une des revendications 1 à 10, **caractérisée en ce qu'**elle contient de la taurine.

12. Boisson selon l'une des revendications 1 à 10, **caractérisée en ce qu'**elle contient des sels minéraux, des macroéléments et/ou des oligoéléments.

13. Composition sous forme de solide, de poudre ou de gel, formulée pour se dissoudre dans un liquide, par exemple comprimés effervescents, pour la fabrication d'une boisson à usage humain selon l'une des revendications 1 à 12, laquelle composition contient au moins les ingrédients : acide acétique, acide propanoïque et acide butanoïque ainsi que les ingrédients : acide méthylpropanoïque et/ou acide méthylbutanoïque.

14. Utilisation d'une boisson selon l'une des revendications 1 à 12 pour augmenter la sécrétion de testostérone chez l'être humain, en particulier chez l'homme.

15. Médicament sous forme de boisson selon l'une des revendications 1 à 12 ou de composition selon la revendication 13 pour la prévention et/ou le traitement de la baisse du taux de testostérone.
